# EUROPÄISCHE PATENTSCHRIFT

(11) **EP 0 484 271 B1**
(45) Veröffentlichungstag und Bekanntmachung des Hinweises auf die Patenterteilung: **19.10.1994**
(21) Anmeldenummer: 91810727.7
(22) Anmeldetag: 11.09.1991
(51) Int. Cl.: C07C 67/31, C07C 69/68, C07C 69/732

(54) **Verfahren zur Herstellung von optisch aktiven aliphatischen Hydroxycarbonsäureestern**
Process for the preparation of optically active aliphatic hydroxycarboxylic acid esters
Procédé pour la préparation d'esters optiquement actifs d'acides hydroxy carboxyliques aliphatiques

(30) Priorität: 02.11.1990 CH 3490/90
(43) Veröffentlichungstag der Anmeldung: 06.05.1992
(73) Patentinhaber: CIBA-GEIGY AG, 4002 Basel (CH)
(72) Erfinder: Blaser, Hans-Ulrich, Dr., CH-4107 Ettingen (CH); Pittelkow, Ulrich, W-7888 Rheinfelden (CH); Spindler, Felix, Dr., CH-4656 Starrkirch-Will (CH)

(56) Entgegenhaltungen:
- PATENT ABSTRACTS OF JAPAN, unexamined applications, C Sektion, Band 2, Nr. 140,18. November 1978 THE PATENT OFFICE JAPANESE GOVERNMENT Seite 3095 C 78
- PATENT ABSTRACTS OF JAPAN, unexamined applications, C Sektion, Band 2, Nr. 140,18. November 1978 THE PATENT OFFICE JAPANESE GOVERNMENT Seite 3096 C 78

## Beschreibung

Die Erfindung betrifft ein Verfahren zur Herstellung von enantiomeren aliphatischen α-Hydroxycarbonsäureestern durch katalytische Hydrierung von prochiralen aliphatischen α-Ketocarbonsäureestern in Gegenwart von Rhodiumkomplexen mit einem chiralen Diphosphinliganden, der zusammen mit dem Rhodiumatom einen Fünfring bildet.

Die enantioselektive homogene Hydrierung von prochiralen α-Ketocarbonsäureestern in Gegenwart von Rhodiumkomplexen mit chiralen Diphosphinliganden ist bekannt und zum Beispiel von B. Heil et al. in Homogeneous Catalysis with Metal Phosphine Complexes, Plenum Press, New York, Seiten 324-332 (1983) oder von B. Heil in Wissenschaftliche Zeitschrift THLM 27, Seiten 746-750 (1985) beschrieben. In diesen Publikationen wird ausdrücklich darauf hingewiesen, dass chirale bidentate Bis(diphenylphosphin)-Liganden, die mit dem Rhodiumatom einen Fünfring bilden, für die Reduktion von Ketonen nicht geeignet sind.

Es wurde nun gefunden, dass man mit solchen einen Fünfring enthaltenden Rhodiumkatalysatoren hohe chemische Umsätze und hohe Enantioselektivitäten erzielen kann, wenn man als Ketone aliphatische α-Ketocarbonsäureester verwendet und der Rhodiumkatalysator ein bicyclisches 1,2-Diphosphin enthält.

Gegenstand der Erfindung ist ein Verfahren zur Herstellung von optisch aktiven aliphatischen α-Hydroxycarbonsäureestern der Formel I

R-C*H(OH)CO₂R₁ (I),

worin R für lineares oder verzweigtes C₁-C₁₂-Alkyl steht, das unsubstituiert oder mit Halogen, -OH, -CO₂R₁, -CO₂N(R₂)₂, (R₂)₂N-, C₁-C₆-Alkoxy oder -Alkylthio, C₄-C₈-Cycloalkyl, C₄-C₈-Cycloalkoxy, C₄-C₈-Cycloalkylthio, Phenyl, Naphthyl, Phenyloxy, Naphthyloxy, Phenylthio oder Naphthylthio substituiert ist, und wobei die Substituenten Cycloakyl, Cycloalkoxy, Cycloalkylthio, Phenyl, Naphthyl, Phenyloxy, Naphthyloxy, Phenylthio und Naphthylthio unsubstituiert oder mit Halogen, -OH, -CO₂R₁, -CO₂N(R₂)₂, (R₂)₂N-, C₁-C₆-Alkoxy oder -Alkylthio substituiert sind; R₁ C₁-C₆-Alkyl, Phenyl oder Benzyl bedeutet; beide R₂ unabhängig voneinander C₁-C₆-Alkyl, Phenyl, Benzyl oder zusammen Tetramethylen, Pentamethylen, 3-Oxa-1,5-pentylen oder N-(C₁-C₆-Alkyl)-3-aza-1,5-pentylen darstellen, und * für überwiegend R- oder S-Konfiguration steht, durch Hydrierung von α-Ketocarbonsäureestern der Formel II

R-C(=O)CO₂R₁ (II),

worin R und R₁ die zuvor angegebenen Bedeutungen haben, in Abwesenheit oder in Gegenwart eines inerten Lösungsmittels bei einem Druck von 0,1 bis 15 MPa und Temperaturen von -20 °C bis 100 °C sowie in Gegenwart katalytischer Mengen eines Rhodiumkomplexes mit chiralen Diphosphinliganden, das dadurch gekennzeichnet ist, dass der Rhodiumkomplex der Formel III entspricht,

[XRhYZ] (III),

worin X für zwei C₂-C₁₂-Olefine oder ein C₅-C₁₂-Dien steht, Z für Cl, Br oder I steht, und Y ein chirales Diphosphin der Formel IV

(R₃)₂P-Q-P(R₃)₂ (IV)

bedeutet, worin R₃ Phenyl, C₁-C₆-Alkylphenyl oder C₁-C₆-Alkoxyphenyl darstellt, und Q für [2,2,1]-Bicyloheptan-1,2-ylen oder [2,2,1]-Bicyclohept-4,5-en-1,2-ylen steht.

R enthält als Alkyl bevorzugt 1 bis 6 C-Atome und besonders bevorzugt 1 bis 4 C-Atome. Beispiele für Alkyl sind Methyl, Ethyl, und die Isomeren von Propyl, Butyl, Pentyl, Hexyl, Heptyl, Octyl, Nonyl, Decyl, Undecyl und Dodecyl.

Der Rest R und die Substituenten Cyloalkyl, Cycloalkoxy, Cycloalkylthio, Phenyl, Naphthyl, Phenyloxy, Naphthyloxy, Phenylthio und Naphthylthio können mit gleichen oder verschiedenen Substituenten ein- oder mehrfach, vorzugsweise ein- bis dreifach substituiert sein. Geeignete Substituenten für R und die genannten Substituenten sind neben -OH, als Halogen -F, -Cl und -Br;
als Alkoxy bevorzugt C₁-C₄-Alkoxy, zum Beispiel Methoxy, Ethoxy, n- oder i-Propoxy, n-, i- oder t-Butoxy, Pentoxy und Hexoxy,
als Alkylthio bevorzugt C₁-C₄-Alkylthio, zum Beispiel Methylthio und Ethylthio,
als Cycloalkyl bevorzugt Cyclopentyl und Cyclohexyl,
als Cycloalkoxy bevorzugt Cyclopentyloxy und Cyclohexyloxy,
als Cycloalkylthio bevorzugt Cyclopentylthio und Cyclohexylthio,
als -CO₂R₁ bevorzugt -CO₂CH₃ und -CO₂C₂H₅,
als -CO₂N(R₂)₂ bevorzugt -CO₂N(CH₃)₂ und -CO₂N(C₂H₅)_{2,}
und als (R₂)₂N- bevorzugt Methylamino, Ethylamino, Dimethylamino und Diethylamino.

Bei R₁ und R₂ kann es sich in der Bedeutung von Alkyl bevorzugt um Methyl, Ethyl, n- oder i-Propyl, n-, i- oder t-Butyl, Pentyl oder Hexyl handeln.

In einer bevorzugten Ausführungsform stellt R lineares oder verzweigtes C₁-C₄-Alkyl und besonders C₁- oder C₂-Alkyl dar, das unsubstituiert oder mit -F, -Cl, -Br, -OH, -CN, -CO₂R₁, -CO₂N(R₂)₂, (R₂)₂N-, C₁-C₄-Alkoxy, C₄-C₈-Cycloalkyl, Phenyl, Phenyloxy oder Phenylthio substituiert ist, und wobei die Substituenten Cycloalkyl, Phenyl, Phenyloxy und Phenylthio unsubstituiert oder mit -F, -Cl, -Br, -OH, -CN, -CO₂R₁, -CO₂N(R₂)₂, (R₂)₂N- oder C₁-C₄-Alkoxy substituiert sind; R₁ C₁-C₄-Alkyl, Phenyl oder Benzyl bedeutet; beide R₂ unabhängig voneinander H, C₁-C₄-Alkyl, Phenyl, Benzyl oder zusammen Tetramethylen, Pentamethylen, 3-Oxa-1,5-pentylen oder N-(C₁-C₄-Alkyl)-3-aza-1,5-pentylen darstellen. Ganz besonders bevorzugt steht R für C₁-C₄-Alkyl, Benzyl oder β-Phenylethyl.

In einer weiteren bevorzugten Ausführungsform stellen R₁ bevorzugt C₁-C₄-Alkyl, Phenyl oder Benzyl und beide R₂ unabhängig voneinander bevorzugt Methyl, Ethyl, n-Propyl, n-Butyl, oder zusammen Tetramethylen oder Pentamethylen dar.

X in der Bedeutung als Olefin enthält bevorzugt 2 bis 6 und besonders bevorzugt 2 bis 4 C-Atome. Besonders bevorzugt ist Ethylen; weitere Beispiele sind Propen und 1-Buten. X enthält als Dien bevorzugt 5 bis 8 C-Atome, wobei es sich um ein offenkettiges oder mono- oder bicyclisches Dien handeln kann. Die beiden Olefingruppen des Diens sind bevorzugt durch ein oder zwei CH₂-Gruppen verbunden.Beispiele sind 1,3-Pentadien, Cyclopentadien, 1,4-Hexadien, 1,4.Cyclohexadien, 1,4- oder 1,5-Heptadien, 1,4- oder 1,5-Cycloheptadien, 1,4- oder 1,5-Octadien, 1,4- oder 1,5-Cyclooctadien, Norbornadien. Bevorzugt stellt X zwei Ethylen, 1,4-Hexadien, 1,5-Cyclooctadien oder Norbornadien dar.

In Formel III steht Z bevorzugt für Cl oder Br.

In Formel IV bedeutet R₃ bevorzugt Phenyl, Methylphenyl, Dimethylphenyl, Methoxyphenyl oder Dimethoxyphenyl. Besonders bevorzugt stellt R₃ Phenyl dar.

Das Diphosphin der Formel IV ist besonders bevorzugt 1,2-Bis-diphenylphosphino-[2,2,1]-bicyclohept-4,5-en, das entweder in der R- oder S-Form vorliegt. Dieses Diphosphin wird auch als (+)-NORPHOS bzw. (-)-NORPHOS bezeichnet.

Die Verbindungen der Formel II sind bekannt und teilweise käuflich, oder sie können nach analogen Verfahren hergestellt werden.Die Verbindungen der Formeln III sind bekannt, siehe zum Beispiel EP-A-0 302 021. Diese Rhodiumkatalysatoren werden im allgemeinen vor der Reaktion in situ hergestellt und direkt verwendet. Die Rhodiumkatalysatoren werden bevorzugt in Mengen von 0,01 bis 5, besonders bevorzugt 0,05 bis 2 Mol-% eingesetzt, bezogen auf die Verbindungen der Formel II. Das Molverhältnis von Verbindungen der Formel II zu Verbindungen der Formel III kann 10000 bis 20, bevorzugt 2000 bis 50 betragen.

Das erfindungsgemässe Verfahren wird bevorzugt bei Temperaturen von 0 °C bis 80 °C und bevorzugt bei einem Wasserstoffdruck von 2 bis 10 MPa durchgeführt

Die Reaktion kann ohne oder in Gegenwart eines inerten Lösungsmittels durchgeführt werden. Geeignete Lösungsmittel, die alleine oder als Mischung eingesetzt werden können, sind zum Beispiel Alkohole wie z.B. Methanol, Ethanol, Propanol, Butanol, Pentanol und Hexanol. Geeignete Mischungslösungsmittel sind zum Beispiel aliphatische und aromatische Kohlenwasserstoffe, wie z.B. Pentan, Hexan, Cyclohexan, Methylcyclohexan, Benzol, Toluol und Xylol; Ether wie z.B. Diethylether, Diethylenglykoldimethylether, Ethylenglykoldimethylether, Tetrahydrofuran und Dioxan; Ester und Lactone wie z.B. Essigsäureethylester, Butyrolacton oder Valerolacton; Carbonsäureamide und Lactame wie z.B. Dimethylformamid, Dimethylacetamid und N-Methylpyrrolidon. Bevorzugte Mischungen sind solche aus Alkoholen und aromatischen Kohlenwasserstoffen, zum Beispiel Methanol/Benzol oder Methanol/Toluol. Bevorzugt wird als Lösungsmittel Methanol alleine oder im Gemisch mit Benzol oder Toluol verwendet.

Eine besonders bevorzugte Ausführungsform ist dadurch gekennzeichnet, dass man α-Ketopropionsäureethylester oder Brenztraubensäureethylester einsetzt, ein Alkanol als Lösungsmittel verwendet, und die Hydrierung bei einem Druck von 2 bis 10 MPa und einer Temperatur von 0 bis 80 °C durchführt.

Die Verbindungen der Formel I sind wertvolle Zwischenprodukte zur Herstellung von biologisch aktiven Wirkstoffen besonders für die Bereiche der Agrarchemikalien und der Pharmazeutika. Wenn R in Formel I β-Phenylethyl bedeutet, können z. B. daraus ACE-Inhibitoren hergestellt werden (siehe EP-A-0 206 993). Allgemein sind die Verbindungen der Formel I Zwischenprodukte zur Herstellung von optisch aktiven α-Aminosäuren, die zum Beispiel in der Peptidsynthese Verwendung finden (siehe R. M. Williams, Synthesis of Optically Active α-Amino Acids, Pergamon Press, New York, Seite 196 (1990).

Die nachfolgenden Beispiele erläutern die Erfindung näher.

Beispiel 1:1,35 g (11,64 mmol) Brenztraubensäuremethylester (Substrat) werden in einen 50 ml Stahlautoklaven eingetragen, der anschliessend fünfmal mit Argon gespült wird. Danach wird mittels einer Stahlkapillare eine Katalysatorlösung zugegeben, die aus 53,3 mg (0,116 mmol) [Rh(Norbornadien)Cl]₂, 117,8 mg (0,255 mmol) (+)-NORPHOS und je 10 ml Methanol und Toluol besteht. Das Molverhältnis Substrat/Katalysator beträgt 100. Dann werden in drei Spülzyklen 1 MPa Wasserstoff aufgepresst und der Wasserstoffdruck auf 2 MPa eingestellt. Die Reaktionsmischung wird bei 25 °C 13 Stunden lang gerührt und darauf in einen Kolben übergeführt und das Lösungsmittel am Rotationsverdampfer entfernt. Bei einem chemischen Umsatz von 100 % erhält man α-Hydroxypropionsäure mit einem Enantiomerenüberschuss (ee) von 89 %.
Bestimmung des Umsatzes: Gaschromatographisch mit Varian® 3700, Säule OV 101, 2 m, T: 50 °C isotherm.
Bestimmung der optischen Ausbeute: Gaschromatographisch nach Derivatisierung des Rohproduktes mit Isopropylisocyanat; Säule Chirasil-L-Val, 50 m, T: 150 °C, isotherm.

Beispiel 2: Es wird analog Beispiel 1 verfahren und die Reaktonsbedingungen wie folgt abgewandelt:
4,0 g Brenztraubensäureethylester, Molverhältnis Substrat/Katalysator 800, Druck 10 MPa, Temperatur 35 °C, Reaktionszeit 17 Stunden. Der Umsatz ist 100 %, ee: 80 %.

Beispiel 3: Es wird analog Beispiel 1 verfahren und die Reaktionsbedingungen wie folgt abgewandelt:
2,4 g 1-Phenyl-α-Ketobuttersäureethylester, Molverhältnis Substrat/Katalysator 50, Lösungsmittel Methanol, Druck 2 MPa, Temperatur 22 °C, Reaktionszeit 2 Stunden. Der Umsatz beträgt 80 %, ee: 95,6 %.

Beispiel 4: Es wird analog Beispiel 3 verfahren und die Reaktionsbedingungen wie folgt abgewandelt:
1,0 g 1-Phenyl-α-Ketobuttersäureethylester, Molverhältnis Substrat/Katalysator 200, Druck 10 MPa, Temperatur 30 °C, Reaktionszeit 20 Stunden. Der Umsatz beträgt 100 %, ee: 91,4 %.

Beispiele 5-9: Es wird analog Beispiel 1 verfahren und die Reaktionsbedingungen wie folgt abgewandelt:
2,17 g (10,5 mMol) Brenztraubensäureethylester, 22 mg (0,048 mMol) [Rh(Norbornadien)Cl]₂, 50 mg (0,11 mMol) (+)-NORPHOS, 25 °C, 10 MPa, 30 ml Lösungsmittel. Weitere Angaben befinden sich in der nachfolgenden Tabelle.

| Beispiel | Lösungsmittel | Hydrierzeit (Stunden) | Umsatz (%) | ee* (%) |
|---|---|---|---|---|
| 5 | Isopropanol | 17 | 98,5 | 85 |
| 6 | n-Butanol | 17 | 79 | 84 |
| 7 | Tetrahydrofuran/Methanol (1:1) | 19,5 | 85 | 90,5 |
| 8 | Ethanol/Essigsäureethylester (1:1) | 20 | 88 | 80,5 |
| 9 | Methanol/Essigsäureethylester (1:1) | 22,5 | 99 | 89 |

| | | | | |
|---|---|---|---|---|
| *) Konfiguration S. | | | | |

Beispiel 10: Es wird analog Beispiel 1 verfahren und die Reaktionsbedingungen wie folgt abgewandelt:
4g 4-Phenyl-α-ketobuttersäureethylester, Verhältnis Substrat:Katalysator 200, Lösungsmittel Ethanol, Druck 10 MPa, Temperatur 30 °C, Hydrierzeit 18 Stunden. Der Umsatz beträgt 98 %; ee 87 % (S).

## Patentansprüche

1. Verfahren zur Herstellung von optisch aktiven aliphatischen α-Hydroxycarbonsäureestern der Formel I
R-C*H(OH)CO₂R₁ (I),
worin R für lineares oder verzweigtes C₁-C₁₂-Alkyl steht, das unsubstituiert oder mit Halogen, -OH, -CO₂R₁, -CO₂N(R₂)₂, (R₂)₂N-, C₁-C₆-Alkoxy oder -Alkylthio, C₄-C₈-Cycloalkyl, C₄-C₈-Cycloalkoxy, C₄-C₈-Cycloalkylthio, Phenyl, Naphthyl, Phenyloxy, Naphthyloxy, Phenylthio oder Naphthylthio substituiert ist, und wobei die Substituenten Cycloakyl, Cycloalkoxy, Cycloalkylthio, Phenyl, Naphthyl, Phenyloxy, Naphthyloxy, Phenylthio und Naphthylthio unsubstituiert oder mit Halogen, -OH, -CO₂R₁, -CO₂N(R₂)₂, (R₂)₂N-, C₁-C₆-Alkoxy oder -Alkylthio substituiert sind; R₁ C₁-C₆-Alkyl, Phenyl oder Benzyl bedeutet; beide R₂ unabhängig voneinander C₁-C₆-Alkyl, Phenyl, Benzyl oder zusammen Tetramethylen, Pentamethylen, 3-Oxa-1,5-pentylen oder N-(C₁-C₆-Alkyl)-3-aza-1,5-pentylen darstellen, und * für überwiegend R- oder S-Konfiguration steht, durch Hydrierung von α-Ketocarbonsäureestern der Formel II
R-C(=O)CO₂R₁ (II),
worin R und R₁ die zuvor angegebenen Bedeutungen haben, in Abwesenheit oder in Gegenwart eines inerten Lösungsmittels bei einem Druck von 0,1 bis 15 MPa und Temperaturen von -20 °C bis 100 °C sowie in Gegenwart katalytischer Mengen eines Rhodiumkomplexes mit chiralen Diphosphinliganden, dadurch gekennzeichnet dass der Rhodiumkomplex den Formeln III entspricht,
[XRhYZ] (III),
worin X für zwei C₂-C₁₂-Olefine oder ein C₅-C₁₂-Dien steht, Z für Cl, Br oder I steht, und Y ein chirales Diphosphin der Formel IV
(R₃)₂P-Q-P(R₃)₂ (IV)
bedeutet, worin R₃ Phenyl, C₁-C₆-Alkylphenyl oder C₁-C₆-Alkoxyphenyl darstellt, und Q für [2,2,1]-Bicyloheptan-1,2-ylen oder [2,2,1]-Bicyclohept-4,5-en-1,2-ylen steht.

2. Verfahren gemäss Anspruch 1, dadurch gekennzeichnet, dass die Reaktionstemperatur 0 °C bis 80 °C beträgt.

3. Verfahren gemäss Anspruch 1, dadurch gekennzeichnet, dass es bei einem Druck von 2 bis 10 MPa durchgeführt wird.

4. Verfahren gemäss Anspruch 1, dadurch gekennzeichnet, dass R in Formel I für C₁-C₆-Alkyl steht, das unsubstituiert oder wie in Anspruch 1 definiert substituiert ist.

5. Verfahren gemäss Anspruch 1, dadurch gekennzeichnet, dass R₁ C₁-C₄-Alkyl, Phenyl oder Benzyl darstellt.

6. Verfahren gemäss Anspruch 1, dadurch gekennzeichnet, dass beide R₂ unabhängig voneinander Methyl, Ethyl, n-Propyl, n-Butyl oder zusammen Tetramethylen oder Pentamethylen darstellen.

7. Verfahren gemäss Anspruch 1, dadurch gekennzeichnet, dass R lineares oder verzweigtes C₁-C₄-Alkyl darstellt, das unsubstituiert oder mit -F, -Cl, -Br, -OH, -CO₂R₁, -CO₂N(R₂)₂, (R₂)₂N-, C₁-C₄-Alkoxy, C₄-C₈-Cycloalkyl, Phenyl, Phenyloxy oder Phenylthio substituiert ist, und wobei die Substituenten Cycloalkyl, Phenyl, Phenyloxy und Phenylthio unsubstituiert oder mit -F, -Cl, -Br, -OH, -CN, -CO₂R₁, -CO₂N(R₂)₂, (R₂)₂N- oder C₁-C₄-Alkoxy substituiert sind; R₁ C₁-C₄-Alkyl, Phenyl oder Benzyl bedeutet; beide R₂ unabhängig voneinander C₁-C₄-Alkyl, Phenyl, Benzyl oder zusammen Tetramethylen, Pentamethylen, 3-Oxa-1,5-pentylen oder N-(C₁-C₄-Alkyl)-3-aza-1,5-pentylen darstellen.

8. Verfahren gemäss Anspruch 1, dadurch gekennzeichnet, dass X in den Formeln III ein C₂-C₆-Olefin oder ein offenkettiges oder mono- oder bicyclisches C₅-C₈-Dien bedeutet.

9. Verfahren gemäss Anspruch 8, dadurch gekennzeichnet, dass X zwei Ethylen, 1,4-Hexadien, 1,5-Cyclooctadien oder Norbornadien darstellt.

10. Verfahren gemäss Anspruch 1, dadurch gekennzeichnet, dass in Formel III Z für Cl oder Br steht.

11. Verfahren gemäss Anspruch 1, dadurch gekennzeichnet, dass R₃ in Formel IV Phenyl, Methylphenyl, Dimethylphenyl, Methoxyphenyl oder Dimethoxyphenyl bedeutet.

12. Verfahren gemäss Anspruch 1, dadurch gekennzeichnet, dass das Diphosphin der Formel IV 1,2-Bis-diphenylphosphino-[2,2,1]-bicyclohept-4,5-en darstellt, das entweder in der R- oder S-Form vorliegt.

13. Verfahren gemäss Anspruch 1, dadurch gekennzeichnet, dass der Rhodiumkatalysator in einer Menge von 0,01 bis 5 Mol-% eingesetzt wird, bezogen auf die Verbindungen der Formel II.

14. Verfahren gemäss Anspruch 1, dadurch gekennzeichnet, dass man als Lösungsmittel Methanol alleine oder im Gemisch mit Benzol oder Toluol verwendet.

15. Verfahren gemäss Anspruch 1, dadurch gekennzeichnet, dass man α-Ketopropionsäureethylester oder Brenztraubensäureethylester einsetzt, ein Alkanol als Lösungsmittel verwendet, und die Hydrierung bei einem Druck von 2 bis 10 MPa und einer Temperatur von 0 bis 80 °C durchführt.

## Claims

1. A process for the preparation of an optically active aliphatic α-hydroxycarboxylic acid ester of formula I
R-C*H(OH)CO₂R₁ (I),
wherein R is linear or branched C₁-C₁₂alkyl which is unsubstituted or substituted by halogen, -OH, -CO₂R₁, -CO₂N(R₂)₂, (R₂)₂N-, C₁-C₆alkoxy or C₁-C₆alkylthio, C₄-C₈cycloalkyl, C₄-C₈cycloalkoxy, C₄-C₈cycloalkylthio, phenyl, naphthyl, phenoxy, naphthoxy, phenylthio or naphthylthio, and the substituents cycloalkyl, cycloalkoxy, cycloalkylthio, phenyl, naphthyl, phenoxy, naphthoxy, phenylthio and naphthylthio are themselves unsubstituted or substituted by halogen, -OH, -CO₂R₁, -CO₂N(R₂)₂, (R₂)₂N-, C₁-C₆alkoxy or C₁-C₆alkylthio; R₁ is C₁-C₆alkyl, phenyl or benzyl; both substituents R₂ are each independently of the other C₁-C₆alkyl, phenyl, benzyl or, when taken together, are tetramethylene, pentamethylene, 3-oxa-1,5-pentylene or N-(C₁-C₆alkyl)-3-aza-1,5-pentylene, and * denotes the predominantly R- or S-configuration, by hydrogenation of an α-ketocarboxylic acid ester of formula II
R-C(=O)CO₂R₁ (II),
wherein R and R₁ are as defined above, in the absence or presence of an inert solvent at a pressure of 0.1 to 15 MPa and at a temperature of from -20°C to 100°C, and also in the presence of catalytic amounts of a rhodium complex with chiral diphosphine ligands, which rhodium complex has the formula III
[XRhYZ] (III),
wherein X is two C₂-C₁₂olefins or a C₅-C₁₂diene, Z is Cl, Br or I, and Y is a chiral diphosphine of formula IV
(R₃)₂P-Q-P(R₃)₂ (IV)
wherein R₃ is phenyl, C₁-C₆alkylphenyl or C₁-C₆alkoxyphenyl, and Q is bicyclo[2.2.1]heptane-1,2-diyl or bicyclo[2.2.1]hept-4-ene-1,2-diyl.

2. A process according to claim 1, wherein the reaction temperature is from 0°C to 80°C.

3. A process according to claim 1, which is carried out at a pressure of from 2 to 10 MPa.

4. A process according to claim 1, wherein R in formula I is C₁-C₆alkyl which is unsubstituted or substituted as defined in claim 1.

5. A process according to claim 1, wherein R₁ is C₁-C₄alkyl, phenyl or benzyl.

6. A process according to claim 1, wherein both substituents R₂ are each independently of the other methyl, ethyl, n-propyl or n-butyl or, when taken together, are tetramethylene or pentamethylene.

7. A process according to claim 1, wherein R is linear or branched C₁-C₄alkyl which is unsubstituted or substituted by -F, -Cl, -Br, -OH, -CO₂R₁, -CO₂N(R₂)₂, (R₂)₂N-, C₁-C₄alkoxy, C₄-C₈cycloalkyl, phenyl, phenoxy or phenylthio, and the substituents cycloalkyl, phenyl, phenoxy and phenylthio are themselves unsubstituted or substituted by -F, -Cl, -Br, -OH, -CN, -CO₂R₁, -CO₂N(R₂)₂, (R₂)₂N- or C₁-C₄alkoxy; R₁ is C₁-C₄alkyl, phenyl or benzyl; both substituents R₂ are each independently of the other C₁-C₄alkyl, phenyl or benzyl, or, when taken together, are tetramethylene, pentamethylene, 3-oxa-1,5-pentylene or N-(C₁-C₄alkyl)-3-aza-1,5-pentylene.

8. A process according to claim 1, wherein X in formula III is a C₂-C₆olefin or an open-chain or mono- or bicyclic C₅-C₈diene.

9. A process according to claim 8, wherein X is two ethylenes, 1,4-hexadiene, 1,5-cyclooctadiene or norbornadiene.

10. A process according to claim 1, wherein Z in formula III is Cl or Br.

11. A process according to claim 1, wherein R₃ in formula IV is phenyl, methylphenyl, dimethylphenyl, methoxyphenyl or dimethoxyphenyl.

12. A process according to claim 1, wherein the diphosphine of formula IV is 1,2-bis(diphenylphosphino)bicyclo[2.2.1]hept-4-ene, which is either in the R- or S-form.

13. A process according to claim 1, wherein the rhodium catalyst is used in an amount of from 0.01 to 5 mol %, based on the compounds of formula II.

14. A process according to claim 1, wherein the solvent used is methanol by itself or a mixture of methanol with benzene or toluene.

15. A process according to claim 1, which comprises using ethyl α-ketopropionate or ethyl pyruvate, an alkanol as solvent, and carrying out the hydrogenation under a pressure of from 2 to 10 MPa and at a temperature of from 0 to 80°C.

## Revendications

1. Procédé pour la préparation d'esters α-hydroxycarboxyliques aliphatiques optiquement actifs de formule I :
R-C*H(OH)CO₂R₁ (I),
où R représente un alkyle en C₁-C₁₂ linéaire ou ramifié, qui est non substitué ou substitué par des halogènes, -OH, -CO₂R₁, -CO₂N(R₂)₂, (R₂)₂N-, (alcoxy en C₁-C₆)- ou (alkyle en C₁-C₆)-thio, cycloalkyle en C₄-C₈, cycloalcoxy en C₄-C₈, (cycloalkyle en C₄-C₈)-thio, phényle, naphtyle, phényloxy, naphtyloxy, phénylthio ou naphtylthio, les substituants cycloalkyle, cycloalcoxy, cycloalkylthio, phényle, naphtyle, phényloxy, naphtyloxy, phénylthio et naphtylthio étant non substitués ou substitués par des halogènes, -OH, -CO₂R₁, -CO₂N(R₂)₂, (R₂)₂N-, (alcoxy en C₁-C₆)- ou (alkyle en C₁-C₆)-thio ; R₁ signifie un alkyle en C₁-C₆, phényle ou benzyle ; les deux radicaux R₂, indépendamment l'un de l'autre, représentent les alkyle en C₁-C₆, phényle, benzyle ou, ensemble, les tétraméthylène, pentaméthylène, 3-oxa-1,5-pentylène ou N-(alkyle en C₁-C₆)-3-aza-1,5-pentylène et * représente une configuration à prédominance R ou S, par hydrogénation des esters d'acides α-cétocarboxyliques de formule II :
R-C(=O)CO₂R₁ (II),
où R et R₁ ont les significations données précédemment, en l'absence ou en présence d'un solvant inerte, sous une pression de 0,1 à 15 MPa et à des températures de - 20 à 100 °C, ainsi qu'en présence de quantités catalytiques d'un complexe de rhodium comportant des ligands de type diphosphine chirale, caractérisé en ce que le complexe de rhodium correspond à la formule III :
[XRhYZ] (III),
où X représente deux oléfines en C₂-C₁₂ ou un diène en C₅-C₁₂, Z représente Cl, Br ou I et Y signifie une diphosphine chirale de formule IV :
(R₃)₂P-Q-P(R₃)₂ (IV),
où R₃ représente les phényle, (alkyle en C₁-C₆)phényle ou (alcoxy en C₁-C₆)phényle, et Q représente [2,2,1]-bicycloheptan-1,2-ylène ou [2,2,1]-bicyclohept-4,5-èn-1,2-ylène.

2. Procédé selon la revendication 1, caractérisé en ce que la température de réaction est de 0 °C à 80 °C.

3. Procédé selon la revendication 1, caractérisé en ce qu'on met en oeuvre la réaction sous une pression de 2 à 10 MPa.

4. Procédé selon la revendication 1, caractérisé en ce que R dans la formule I représente un alkyle en C₁-C₆, qui est non substitué ou substitué comme il est défini dans la revendication 1.

5. Procédé selon la revendication 1, caractérisé en ce que R₁ représente les alkyle en C₁-C₄, phényle ou benzyle.

6. Procédé selon la revendication 1, caractérisé en ce que les deux radicaux R₂, indépendamment l'un de l'autre, représentent les méthyle, éthyle, n-propyle, n-butyle ou ensemble, les tétraméthylène ou, pentaméthylène.

7. Procédé selon la revendication 1, caractérisé en ce que R représente un alkyle en C₁-C₄ linéaire ou ramifié et particulièrement un alkyle en C₁-ou en C₂-, qui est non substitué ou substitué par -F, -Cl, -Br, -OH, -CN, -CO₂R₁, -CO₂N(R₂)₂, (R₂)₂N-, alcoxy en C₁-C₄, cycloalkyle en C₄-C₈, phényle, phényloxy ou phénylthio, et les substituants cycloalkyle, phényle, phényloxy et phénylthio étant non substitués ou substitués par -F, -Cl, -Br, -OH, -CN, -CO₂R₁, -CO₂N(R₂)₂, (R₂)₂N- ou alcoxy en C₁-C₄ ; R₁ signifie les alkyle en C₁-C₄, phényle ou benzyle ; les deux radicaux R₂, indépendamment l'un de l'autre, représentent H, alkyle en C₁-C₄, phényle, benzyle ou, ensemble, les tétraméthylène, pentaméthylène, 3-oxa-1,5-pentylène ou N-(alkyle en C₁-C₄)-3-aza-1,5-pentylène.

8. Procédé selon la revendication 1, caractérisé en ce que X dans les formules III signifie une oléfine en C₂-C₆ ou un diène en C₅-C₆ à chaîne ouverte ou un diène mono- ou bicyclique.

9. Procédé selon la revendication 8, caractérisé en ce que X représente deux éthylène, 1,4-hexadiène, 1,5-cyclooctadiène ou norbornadiène.

10. Procédé selon la revendication 1, caractérisé en ce que dans la formule III Z représente Cl ou Br.

11. Procédé selon la revendication 1, caractérisé en ce que R₃ dans la formule IV signifie les phényle, méthylphényle, diméthylphényle, méthoxyphényle ou diméthoxyphényle.

12. Procédé selon la revendication 1, caractérisé en ce que la diphosphine de formule IV représente le 1,2-bis-diphénylphosphino-[2,2,1]-bicyclohept-4,5-ène, qui est présent soit sous forme R soit sous forme S.

13. Procédé selon la revendication 1, caractérisé en ce qu'on utilise le catalyseur au rhodium dans une quantité de 0,01 à 5 % molaires, par rapport aux composés de formule II.

14. Procédé selon la revendication 1, caractérisé en ce qu'on utilise en tant que solvant le méthanol seul ou un mélange avec le benzène ou le toluène.

15. Procédé selon la revendication 1, caractérisé en ce qu'on utilise l'ester éthylique de l'acide α-cétopropionique ou l'ester éthylique de l'acide pyruvique, un alcanol en tant que solvant, et on met en oeuvre l'hydrogénation sous une pression de 2 à 10 MPa et à une température de 0 à 80 °C.
